# EUROPEAN PATENT SPECIFICATION

(11) **EP 0 714 265 B1**
(45) Date of publication and mention of the grant of the patent: **19.01.2000**
(21) Application number: 95920357.1
(22) Date of filing: 24.05.1995
(51) Int. Cl.: A61B 6/04

(54) **DEVICE FOR COMPRESSION OF THE LUMBAR SPINE FOR MEDICAL IMAGING PURPOSES**
VORRICHTUNG ZUR KOMPRESSION DER LENDEN-WIRBELSÄULE ZUR MEDIZINISCHEN BILDDARSTELLUNG
DISPOSITIF DE COMPRESSION DE LA COLONNE LOMBAIRE A DES FINS D'IMAGERIE MEDICALE

(30) Priority: 24.05.1994 SE 9401793
(43) Date of publication of application: 05.06.1996
(73) Proprietor: BOHUSLANDSTINGET, 401 50 Göteborg (SE)
(72) Inventor: WILLEN, Jan A. G., S-435 43 Mölnlycke (SE); GAULITZ, Arne, S-427 29 Billdal (SE); DANIELSSON, Barbro, S-437 40 Lindome (SE); NICKLASSON, Thomas, S-439 35 Onsala (SE)
(74) Representative: Roth, Ernst Adolf Michael
(86) International application number: PCT/SE95/00581
(87) International publication number: WO 95/31936

(56) References cited:
- US-A- 3 629 581
- US-A- 4 202 355
- US-A- 4 669 106

## Description

### Technical field

The present invention relates to a device for diagnosing purposes to be used in complementary examination of the lumbar spine at computed tomography or magnetic resonance tomography.

The object of the present invention is to obtain a device, the use of which provides an aid for an adequate and reproducible examination of the lumbar spine in connection with computed tomography and/or magnetic resonance tomography, in particular for the diagnosis of stenosis of the spinal cord canal and nerve structures (spinal stenosis) present therein.

### Background of the invention

Traditionally, the patient is examined in computed tomography and magnetic resonance tomography in relaxed supine position, which means that those structures in connection with the spinal cord canal which are variable, (intervertebral discs, ligaments and small joints) are subject to minimal load. At load the pressure in inter alia the intervertebral discs increases, resulting in a protuberance which in turn diminishes the space in the spinal cord canal and nerve and vessel structures present therein. At examination of the spinal cord canal of the lumbar spine while suspecting spinal stenosis, and using computes tomography or magnetic resonance tomography there is a need for being able to load the lumbar spine in the same way as it occurs at upright standing, simultaneously as the patient is placed in a horizontal position.

It is previously known from US-A-3,629,581 a device for positioning a patients shoulders in connection with an X-ray examination of the spine of a patient, whereby the upper spinal column is pressed down towards the examination table and makes it possible to obtain good X-ray picture of the upper vertebras. Hereby a pressure is applied over the spinal column of the lying patient via two flexible strings provided with handles for the patient, which strings are arranged around a foot plate.

US-A-4,534,076 relates to a device for placing a patient in an upside down position in order to deload a patient's spinal column.

A device according to the preamble of claim 1 is known from US-A-4,202,355 which discloses X-ray grid orthometer used for measuring the leg length of individuals that may have an anatomical leg length imbalance, whereby the device comprises a frame means mountable on a support for adjusting to the length of the legs of a patient whereby the frame comprises two foot plates onto which the patient's feet are placed. Further the X-ray grid orthometer comprises a pair of pulleys and a flexible elongated member arranged as to be engageable at the end portions by a patient for compressing the lumbar spine and legs of the patient pulling with both hands on the flexible member.

There is thus a problem to be solved at the examination of the spinal canal of the lumbar spine.

### Description of the present invention

It has now surprisingly been shown possible to be able to solve this problem by the present invention which comprises a resting surface being part of a patient table, characterized in that it further comprises
a foot plate comprising pressure sensors and being arranged to said resting surface and being arranged to accommodate the feet of a patient, whereby said pressure sensors are arranged to register the pressure of the respective foot on said footplate and to show said pressure on a pressure meter,
a waistcoat arranged to be placed around the body of a patient,
pulling cords arranged between said waistcoat and said foot plate, as well as a straining device arranged to said pulling cords, and arranged to be strained to a predetermined value.

By means of the present invention a controlled load of the lumbar spine can be obtained when the waistcoat applied around a patient's body is drawn using a predetermined force against said foot plate by means of said pulling straining device, against which foot plate the patient's feet are arranged, whereby the pressure sensors indicates the pressure/pulling obtained, and thereby the compression of the lumbar spine as desired.

By means of the compression obtained the space in the spinal canal is reduced and the diagnosis of the spinal stenosis is facilitated and become more safe.

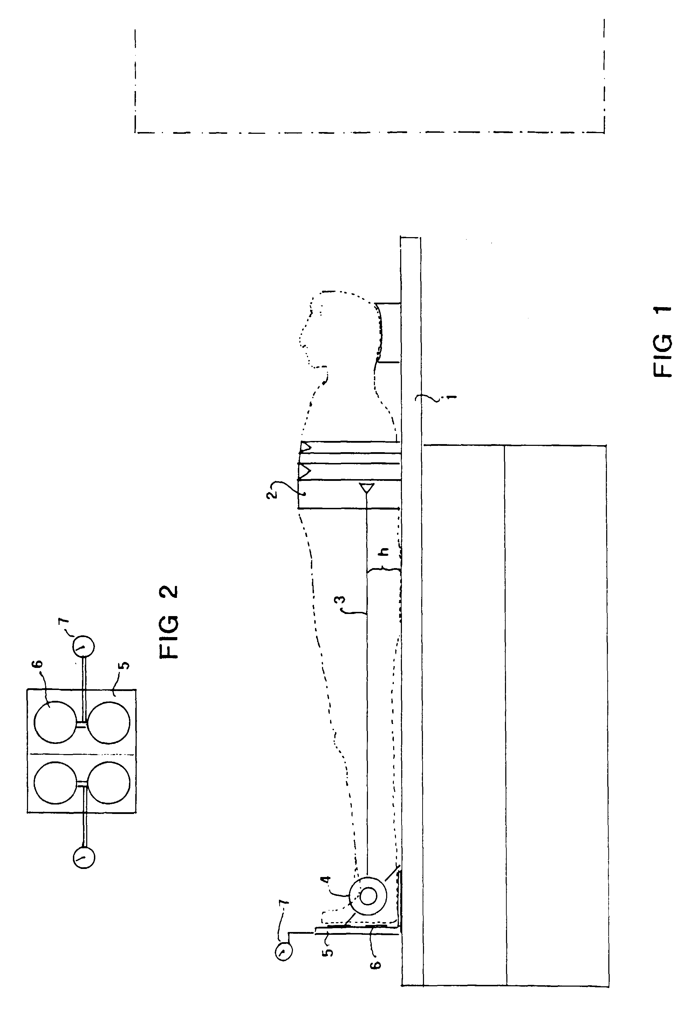

The invention will now be described more in detail with reference to the attached drawing, showing a preferred device of the invention, wherein
FIG. 1 shows a lateral view of one embodiment of a device according to the present invention, and
FIG. 2 shows an embodiment of a foot plate of the device according to FIG. 1.

1 denotes a resting surface of a patient table being suitable for being introduced in a device for computed tomography or magnetic resonance tomography. The computed tomography and the magnetic resonance tomography are known units and are not subject of the present invention and will thus not be described more in detail. 2 denotes a waistcoat, which is arranged to be placed around the upper body of a patient, the thoracic region, whereby it is fastened suitably with the use of cords of the burdock type. The waistcoat 2 is applied firmly, but still comfortably. Pulling cords 3 run on each side of the patient from the waistcoat 2, whereby said cords are placed at a height h above the resting surface. This height h will be closely defined later. The other ends of the pulling cords 3 arc attached to a straining device 4, which in a simple embodiment is a mechanical rolling device, manually or motor driven. The straining device 4 is in turn attached to a foot plate 5 which has such a dimension that the two feet of a patient are accommodated upon this. The foot plate 5 is provided with pressure sensors 6 in the form of air cylinders and are further connected to their respective manometer 7 for the respective foot. The pressure sensors 6 register the pressure of the respective foot on the foot plate 5 and this pressure can be read on the manometer 7.

The height **h** at which the pulling cords are arranged over the resting surface 1, can be varied depending on the constitution of the patient, but also depending on the desired load on the spinal column/lumbar spine.

The pressure sensors 6 may also be connected to a motor which affects the straining of the pulling cords 3, whereby a predetermined, adjusted value can be maintained as the pressure sensors 6 control this motor.

Further the pressure sensors 6 can be arranged to control a pressure cylinder arranged between the pulling cords 3 and the foot plate 5 which pressure cylinder then is straining the cords.

At an examination a patient is placed on his back upon the resting surface 1, the waistcoat 2 is arranged around the thorax of the patient and the pulling cords 3 are connected to the waistcoat 2. The pulling cords 3 are then strained by means of the straining device 4 either manually or by means of a motor, whereby they are strained to predetermined value, most often depending on the weight of the patient, whereby for the average patient this value shall provide a load of about 40 kg on the lumbar spine, i.e., about half of the body weight which is the normal load at upright standing. The pressure sensors 6 determine the pressure and it can be read on the manometers, whether the load is reached, if it is evenly distributed, etc.

The patient is then introduced in the computed tomography or magnetic resonance tomography equipment in order to obtain the desired pictures of the desired region.

## Claims

1. Device to be used in complementary examination of the lumbar spine at computed tomography and magnetic resonance tomography
that comprises a resting surface (1) being part of a patient table,
**characterized** in that it further comprises
a foot plate (5) comprising pressure sensors (6) and being arranged to said resting surface and being arranged to accommodate the feet of a patient, whereby said pressure sensors (6) are arranged to register the pressure of the respective foot on said footplate and to show said pressure on a pressure meter,
a waistcoat (2) arranged to be placed around the body of a patient,
pulling cords (3) arranged between said waistcoat (2) and said foot plate (5), as well as a straining device (4) arranged to said pulling cords (4), and arranged to be strained to a predetermined value.

2. Device according to claim 1,
**characterized** in that the pulling force of the pulling cords (3) is arranged to be regulated manually.

3. Device according to claim 1,
**characterized** in that the pulling force of the pulling cords (3) is arranged to be regulated using a motor.

4. Device according to claim 3,
**characterized** in that the pulling force of the pulling cords (3) is arranged to be regulated using a motor being controlled by said pressure sensors (6).

5. Device according to claim 1,
**characterized** in that the pulling force of the pulling cords (3) is arranged to be regulated by means of a pressure cylinder arranged as a straining device (4) which pressure cylinder is controlled by said pressure sensors (6).

6. Device according to one or more of the preceding claims,
**characterized** in that the heigh (**h**) of the pulling cords (3) above the resting surface (1) is arranged to be variable at the connection to the waistcoat (2).

## Patentansprüche

1. Vorrichtung zur Verwendung bei der komplementären Untersuchung der Wirbelsäule durch Computertomographie und Magnetresonanztomographie, wobei die Vorrichtung eine Stützfläche (1) als Teil eines Patiententisches aufweist, dadurch gekennzeichnet, daß sie außerdem aufweist
eine Fußplatte (5) mit Drucksensoren (6) und angeordnet auf der Stützfläche sowie derart, daß sie die Füsse eines Patienten aufnimmt, wobei die Drucksensoren (6) so angeordnet sind, daß sie den Druck des jeweiligen Fusses auf die Fußplatte zumessen und den Druck eines Druckmeßgeräts anzeigen,
eine Weste (2), die zur Anbringung um den Körper eines Patienten dient,
Zuggurte (3), die zwischen der Weste (2) und der Fußplatte (5) angeordnet sind sowie eine Spannvorrichtung (4), die an den Zugseilen (4) angeordnet und dazu ausgelegt ist, diese auf einen vorbestimmten Wert zu spannen.

2. Vorrichtung nach Anspruch 1, dadurch gekennzeichnet, daß die Zugkraft der Zugseile (6) zur manuellen Einstellung ausgelegt sind.

3. Vorrichtung nach Anspruch 1, dadurch gekennzeichnet, daß die Zugkraft der Zugseile (3) zur Einstellung unter Verwendung eines Motors ausgelegt ist.

4. Vorrichtung nach Anspruch 3, dadurch gekennzeichnet, daß die Zugkraft der Zugseile (3) zur Einstellung unter Verwendung eines Motors ausgelegt ist, der durch die Drucksensoren (6) gesteuert ist.

5. Vorrichtung nach Anspruch 1, dadurch gekennzeichnet, daß die Zugkraft der Zugseile (3) dazu ausgelegt ist, mittels eines Druckzylinders eingestellt zu werden, der als Spannvorrichtung (4) vorgesehen ist, welcher Druckzylinder durch die Drucksensoren (6) gesteuert ist.

6. Vorrichtung nach einem der vorangehenden Ansprüche, dadurch gekennzeichnet, daß die Höhe h der Zugseile (3) über der Stützfläche (1) in Verbindung der Weste (2) einstellbar ist.

## Revendications

1. Dispositif destiné à être utilisé dans l'examen complémentaire de la colonne lombaire par tomographie assistée (ou numérique) ou tomographie de résonance magnétique, qui comprend une surface de support (1) qui fait partie de la table d'examen, ***caractérisé en ce qu'il*** comporte de plus
un cale-pied (5) comprenant des capteurs de pression (6) et qui est monté sur ladite surface de support et est prévu pour recevoir les pieds d'un patient, lesdits capteurs de pression (6) étant prévus pour enregistrer la pression du pied respectif sur ledit cale-pied et pour afficher ladite pression sur un manomètre,
un gilet (2) prévu pour être placé autour du corps d'un patient,
des cordes de traction (3) placées entre ledit gilet (2) et ledit cale-pied (5), ainsi qu'un dispositif de tension (4) associé auxdites cordes de traction (3) et prévu pour être tendu selon une valeur prédéterminée.

2. Dispositif selon la Revendication 1, ***caractérisé en ce que*** la force de traction des cordes de traction (3) est prévue pour être réglée manuellement.

3. Dispositif selon la Revendication 1, ***caractérisé en ce que*** la force de traction des cordes de traction (3) est prévue pour être réglée en utilisant un moteur.

4. Dispositif selon la Revendication 3, ***caractérisé en ce que*** la force de traction des cordes de traction (3) est prévue pour être réglée en utilisant un moteur contrôlé par lesdits capteurs de pression (6).

5. Dispositif selon la Revendication 1, ***caractérisé en ce que*** la force de traction des cordes de traction (3) est prévue pour être réglée au moyen d'un cylindre de pression prévu comme dispositif de tension (4), lequel cylindre de pression est contrôlé par lesdits capteurs de pression (6).

6. Dispositif selon l'une quelconque des Revendications précédentes, ***caractérisé en ce que*** la hauteur (h) des cordes de traction (3) au-dessus de la surface de support (1) est apte à être modifiée au niveau du rattachement au gilet (2).
